# EUROPEAN PATENT APPLICATION

(11) **EP 4 464 791 A1**
(43) Date of publication of application: **20.11.2024**
(21) Application number: 23174025.9
(22) Date of filing: 17.05.2023
(51) Int. Cl.: C12Q 1/6827, G16B 30/10

(54) **NON-INVASIVE IN-VITRO METHOD FOR SOMATIC MUTATION DETECTION**

(71) Applicant: Medicover Biotech Ltd., 2409 Nicosia Egkomi (CY)
(72) Inventor: KOUMBARIS, George, 2565 Lithrodontas (CY); ACHILLEOS, Acilleas, 4045 Limassol (CY); TSANGARAS, Kyriakos, 3085 Limassol (CY); ELIADES, Alexia, 2102 Nicosia (CY); LOIZIDES, Charalambos, 2013 Nicosia (CY); LEMESIOS, Christos, 2460 Pano Deftera (CY); KYPRI, Elena, 2007 Nico (CY); IOANNIDES, Marios, 2416 Nicosia (CY); PATSALIS, Philippos, 2402 Nicosia (CY)
(74) Representative: CH Kilger Anwaltspartnerschaft mbB

(57) **Abstract**

The invention relates to an in-vitro method of detecting a somatic mutation in a human,
(i) providing for a human sample, preferably a blood sample from a subject, more preferably a plasma sample, or a serum sample or a buffy coat sample,
(ii) preparing a first nucleic acid sequencing library from the nucleic acids present in said sample,
(iii) hybridizing one or more TAC oligonucleotides from a first TAC oligonucleotide pool (TAC oligonucleotide-1) to said first nucleic acid library thereby isolating a first subset of library nucleic acid molecules,
(iv) sequencing said first subset of library nucleic acid molecules and comparing the sequences determined to a human reference sequence, thereby creating a first group of putatively informative sequence variants (PISV1),
(v) hybridizing one or more TAC oligonucleotides from a second smaller TAC oligonucleotide pool comprising TAG oligonucleotides (TAC oligonucleotide-2) which are specific for the nucleic acid molecules of said first group of putatively informative sequence variants, to said first library of nucleic acid molecules thereby isolating a second subset of library nucleic acid molecules,
(vi) sequencing said second subset of library nucleic acid molecules and comparing the sequences determined to a human reference sequence, thereby creating a second group of putatively informative sequence variants (PISV2),
(vii) analyzing the putatively informative sequence variants (PISV2) thereby detecting a somatic mutation in the sample from the subject.

Steps (i) to (iv) are also referred to together as step A. Steps (v) to (vii) are also referred to together as step B.

## Description

### FIELD OF THE INVENTION

The invention is in the field of biology, medicine, and chemistry, in particular the field of molecular biology and more in particular in the field of molecular diagnostics. The invention is in particular in the field of in-vitro diagnostics using cell free nucleic acids and DNA. The invention is also in the field of diagnostic kits.

### BACKGROUND OF THE INVENTION

Early detection of cancer and quantification of the risk of cancer recurrence and metastasis, in patients who are undergoing treatment or are in remission, remain one of the major concerns among patients and health care professionals in the field of oncology. Many of the current methods for detecting early stages of cancer or recurrence and metastasis rely heavily on imaging scans (by which time the tumor would have need to have grown to a sufficient size for it to be detected on the scan), therefore resulting in the loss of crucial treatment time.

Minimal Residual Disease (MRD) refers to the very small number of cancer cells remaining in the patient's body during treatment, or after definitive treatment. Evidence suggests that MRD is a significant contributor to recurrence or metastasis, and therefore critical for the prognosis and management of various cancer types. MRD detection detects the molecular relapse of the disease prior to the manifestation of clinical symptoms and, as a result, offers the advantage of early intervention.

The discovery of free fetal DNA (ffDNA) in maternal circulation was a landmark towards the development of Non-invasive Prenatal Testing (NIPT) for chromosomal abnormalities and has opened up new possibilities in the clinical setting (PMID 9529358). However, direct analysis of the limited amount of ffDNA in the presence of an excess of maternal DNA is a great challenge for NIPT of chromosomal abnormalities. The implementation of next-generation sequencing (NGS) technologies in the development of NIPT has revolutionized the field. In 2008, two independent groups demonstrated that NIPT of trisomy 21 could be achieved using next-generation massively parallel shotgun sequencing (MPSS) (PMID 18945714, PMID 18838674). The new era of NIPT for chromosomal abnormalities has opened new possibilities for the implementation of these technologies into clinical practice. Biotechnology companies that are partly or wholly dedicated to the development of NIPT tests have initiated large-scale clinical studies towards their implementation.

More recently, targeted-based NGS approaches for NIPT, in which only specific sequences of interest are sequenced, have been developed. For example, a targeted NIPT approach using TArget Capture Sequences (TACS) for identifying fetal chromosomal abnormalities using a maternal blood sample has been described (PCT Publication WO 2016/189388; US Patent Publication 2016/0340733; Koumbaris, G. et al. (2015) Clinical chemistry, 62(6), pp.848-855.).

Such targeted approaches require significantly less sequencing than the MPSS approaches, since sequencing is only performed on specific loci on the target sequence of interest, rather than across the whole genome. Additional methodologies for NGS-based approaches are still needed, in particular approaches that can target specific sequences of interest, thereby greatly reducing the amount of sequencing needed as compared to whole genome-based approaches, as well as increasing the read-depth of regions of interest, thus enabling detection of low signal to noise ratio regions. In particular, additional methodologies are still needed that allow for genetic aberrations present in diminutive amounts in a sample to be reliably detected.

Circulating tumor DNA (ctDNA) has likewise emerged as a dynamic biomarker for early detection of cancer, or for the assessment of MRD in real time. To date, most approaches for assessing MRD using ctDNA are tumor-informed approaches, which rely on initial genomic profiling of tumor tissue to identify tumor-derived alterations specific for each individual patient. The rationale behind this approach is that knowledge of tumor-specific mutations, specific to each patient, can potentially increase the sensitivity of MRD detection, as said mutations can be subsequently searched for in ctDNA at predefined time-points. However, although extensively used, the tissue-informed approach has several limitations. For example, the tissue derived from the surgical specimens might be inadequate for tissue sequencing due to limited tumor cellularity, having low DNA quality or yield.

Further to these limitations, the surgical specimens may also fail to adequately capture tumor heterogeneity.

An alternative approach for early detection of cancer and assessment of MRD using ctDNA is the tumor-agnostic approach, which relies on non-invasive, plasma-only assay for MRD detection. The tumor-agnostic approach offers several advantages over the tumor-informed approach. Said advantages include a faster turn-around time since solely a single (blood) sample is analyzed, lower processing costs and substantially decreased logistical complexity. Unlike traditional tissue biopsy, the liquid biopsy is non-invasive, easily repeatable, and may offer an informative insight into the tumor burden and treatment response. Furthermore, liquid biopsy may give a more complete molecular profile of the primary tumor, minimizing bias in biopsy findings which often results from sampling bias and intra-tumor heterogeneity.

Current liquid biopsy- based tests, because of their complexity, as well their limited sensitivity and specificity, do not show high enough and could give rise to misleading results.

Currently available assays for detecting ctDNA from patient plasma test for a fixed panel of hotspot or actionable mutations. Given the heterogeneity of cancer, even large generic panels targeting up to more than a hundred genomic loci, might detect only a few mutations from a given individual's primary tumor. Mutations identified in these panels may not be tumor-derived, making such approaches less specific. Likewise, in most available tests, primary tumor tissue and matched normal blood are collected from each patient. Genomic DNA from tumor tissue and buffy coat are extracted, whole-exome sequenced, analyzed, and filtered for patient-specific somatic mutations.

A very particular and preferred main objective of the current invention is MRD surveillance and early detection of cancer, using a tissue-agnostic approach tailored to the patient's cancer biomarker profile, without the need for a tissue biopsy analysis, complex methylation detection assays and/or high volumes of sample availability.

A more general problem to be solved, and solved by the present invention is providing for a non-invasive, in-vitro molecular diagnostic method that avoids the need for a solid tissue biopsy and allows for diagnosis using blood only.

A more general problem to be solved and solved by the present invention is a providing for a non-invasive in-vitro molecular diagnostic disease detection method that avoids the need for a solid tissue biopsy and allows for diagnosis using blood only.

A more general problem to be solved and solved by the present invention is a providing for a non-invasive in-vitro molecular diagnostic cancer detection method that avoids the need for a solid tissue biopsy and allows for diagnosis using blood only.

A further main objective of the current invention in a more particular and preferred embodiment is MRD surveillance and early detection of cancer, using a tissue-agnostic approach tailored to the patient's cancer biomarker profile, without the need for a solid tissue biopsy analysis, complex methylation detection assays and/or high volumes of sample availability.

As will become entirely clear from the following explanations, the invention is very general in kind and specific in its various forms of applications.

### SUMMARY OF THE INVENTION

The inventors have been able to solve the problems outlined above.

The invention relates to an in-vitro method of detecting a somatic mutation in a human,
(i) providing for a human sample, preferably a blood sample from a subject, more preferably a plasma sample, or a serum sample or a buffy coat sample,
(ii) preparing a first nucleic acid sequencing library from the nucleic acids present in said sample,
(iii) hybridizing one or more TAC oligonucleotides from a first TAC oligonucleotide pool (TAC oligonucleotide-1) to said first nucleic acid library thereby isolating a first subset of library nucleic acid molecules,
(iv) sequencing said first subset of library nucleic acid molecules and comparing the sequences determined to a human reference sequence, thereby creating a first group of putatively informative sequence variants (PISV1),
(v) hybridizing one or more TAC oligonucleotides from a second smaller TAC oligonucleotide pool comprising TAC oligonucleotides (TAC oligonucleotide-2) which are specific for the nucleic acid molecules of said first group of putatively informative sequence variants, to said first library of nucleic acid molecules thereby isolating a second subset of library nucleic acid molecules,
(vi) sequencing said second subset of library nucleic acid molecules and comparing the sequences determined to a human reference sequence, thereby creating a second group of putatively informative sequence variants (PISV2),
(vii) analyzing the putatively informative sequence variants (PISV2) thereby detecting a somatic mutation in the sample from the subject.

Steps (i) to (iv) are also referred to together as step A. Steps (v) to (vii) are also referred to together as step B.

Detecting the presence of a somatic mutation herein refers to identifying a mutation in a blood sample from patient that is derived from a somatic mutational event. Detecting herein of course also means identifying which means positively determining it is a somatic mutation and characterizing the nucleotide type or type of mutation.

In one aspect, the invention described herein pertains to a method of determining the presence of cell-free, circulating tumor DNA (ctDNA) nucleic acid molecules, in a sample comprising a plurality of cell-free DNA (cfDNA) nucleic acid molecules. The current invention does not require a substantial sequencing cost for the analysis of a tissue or plasma sample to achieve high accuracy as previously suggested (Jamshidi et al. [2022] Cancer Cell 40,1537-1549; Kotani, D., Oki, E., Nakamura, Y. et al. [2023]. Nat Med 29, 127-134; Reinert T, Henriksen TV, Christensen E, et al. [2019], JAMA Oncol.:5(8):1124-1131; Coombes RC et al. [2019], Clin Cancer Res 25(14):4255-4263). In one aspect, a small number of candidate somatic single nucleotide variant positions are selected for targeted deep re-sequencing to significantly minimize cost.

In one embodiment, said first subset of library nucleic acid molecules of Step A (iii) is amplified and sequenced to obtained at least 10,000 or at least 20,000 or at least 30,000 or at least 50,000 cfDNA nucleic acid molecules per targeted region. In one embodiment, said second subset of library nucleic acid molecules Step B (vi) is amplified and sequenced to obtained at least 100,000 or at least 200,000 or at least 300,000 or at least 500,000 cfDNA nucleic acid molecules per targeted region.

"Cell-free DNA" as used herein, refers to DNA that is not contained within a cell. A sample may comprise cfDNA from normal or healthy cells and/or from cancer cells. Cell-free DNA may be released into the blood or serum through secretion, apoptosis or necrosis. If cfDNA is released from a tumor or cancer cell, it may be called cell-free tumor DNA (cftDNA).

"Nucleic acid" or "Nucleic acid sequence" as used herein, may be used interchangeably, and may refer without being limited to DNA, RNA, genomic DNA, cell-free DNA and/or RNA, tRNA, messenger RNA (mRNA), synthetic DNA and synthetic RNA.

In the context of the present invention, the term "nucleic acid fragments" and "fragmented nucleic acids" can be used interchangeably. In a preferred embodiment of the method, the nucleic acid fragments are circulating cell-free DNA or RNA or circulating cell-free tumor DNA.

In the context of the present invention, the term "subject" refers to animals, preferably mammals, and more preferably to humans or human patients. As used herein, the term "subject" may refer to a subject suffering from or suspected of having a tumor.

"Sample" as used herein, refers to any biological material derived from a subject. A sample can be a liquid sample or solid sample (e.g., a cell or tissue sample). A biological sample can be a bodily fluid, such as blood, plasma, serum, buffy coat, urine, pleural fluid, ascitic fluid, cerebrospinal fluid, saliva, sputum, bronchoalveolar lavage fluid, aspiration fluid from different parts of the body (e.g., thyroid, breast), etc. A biological sample can be a cell-free sample. Thus, the sample can be a liquid biopsy that is obtained non-invasively from a subject's blood sample, comprising cell-free DNA (cfDNA), cell-free tumor DNA (cftDNA), circulating tumor DNA (ctDNA) or circulating cftDNA (ccftDNA), thereby potentially allowing for early detection of cancer prior to the development of a detectable or palpable tumor, or allowing monitoring of disease progression, disease treatment, or disease relapse. The term "sample" can also refer to a stool sample. In one embodiment, the sample is selected from a group consisting of plasma sample, a blood sample, a buffy coat sample, a urine sample, a sputum sample, a cerebrospinal fluid sample, an ascites sample and a pleural fluid sample from a subject having or suspected of having a tumor or has had tumor resection. In one embodiment, the sample or DNA sample is from a tissue sample from a subject having or suspected of having a tumor or a set of malignant cells. In yet another embodiment, the sample is a stool sample.

In the context of the present invention, the terms "tumor", "cancer" or "abnormal" may be used interchangeably. Herein, the terms "cancer" or "tumor" may also comprise of early stage of cancer or advanced cancer, or metastasis. Herein, a "tumor" sample or "abnormal" sample may relate to a sample comprising (cell-free) DNA or RNA originating from a primary tumor or a metastatic tumor. A "normal" sample or "reference" sample may herein relate to a sample comprising only (cell-free) DNA or RNA originating from non-cancerous, healthy or "normal" tissue(s) or cell(s). In the context of the present invention, the terms "normal", "control" or "healthy" or "reference" may be used interchangeably.

A "tumor" herein refers to cancer in general, including but not limited to a solid tumor, an adenoma, blood cancer, liver cancer, lung cancer, pancreatic cancer, prostate cancer, breast cancer, gastric cancer, glioblastoma, colorectal cancer, head and neck cancer, a tumor of an advanced stage of cancer, a benign or malignant tumor, a metastasis or a precancerous tissue.

Hybridization as used herein, refers to the annealing of one or more probes to target nucleotide sequence. Hybridization conditions typically include a temperature that is below the melting temperature of the TAC oligonucleotides, but that avoids non-specific hybridization of the TAC oligonucleotides.

Sensitivity as used in the present disclosure can refer to the number of true positives divided by the sum of the number of true positives and false negatives. Sensitivity can characterize the ability of an assay or method to correctly identify a proportion of the population that truly has a condition. For example, sensitivity can characterize the ability of a method to correctly identify the number of subjects within a population having cancer.

Specificity as used in the present disclosure, can refer to the number of true negatives divided by the sum of the number of true negatives and false positives. Specificity can characterize the ability of an assay or method to correctly identify a proportion of the population that truly does not have a condition. For example, specificity can characterize the ability of a method to correctly identify the number of subjects within a population not having cancer.

The "ratio of good quality to poor quality" aligned cfDNA nucleic acid molecules that support the variant nucleotide, is defined as the number of aligned cfDNA nucleic acid molecules that support the variant nucleotide with mapping quality of 45 or greater, divided by the number of aligned cfDNA nucleic acid molecules that support the variant nucleotide with mapping quality of less than 45 plus 0.5 to account for the cases in which the denominator is 0. In other embodiments of the current invention a mapping quality of 20 or 25 or 30 or 35 or 40 is used.

As used herein, "mapping quality" is defined as the probability that a read is aligned in the wrong place (phred-scaled posterior probability that the mapping position of this read is incorrect).

A log odds likelihood is defined as the logarithm of the probability of a candidate somatic single nucleotide variant to be a true event originating from a tumor tissue over the probability of it to be an artifact/erroneous call.

In a preferred embodiment, preparing the DNA sequencing library comprises the step of including unique molecular identifiers (UMIs) to uniquely tag each molecule and to create UMI families.

"Consensus sequences" are the calculated order of most frequent residues, either nucleotide or amino acid, found at each position in a sequence alignment. It serves as a unified representation of each UMI family, said UMI family being defined as the aligned sequencing reads (cfDNA molecules) having the same start and stop position relative to the reference genome and the same UMI barcodes. In the context of the present invention, the terms "mutation" or "variant nucleotide" may be used interchangeably, and generally refer to a mutation (e.g., a single base pair mismatch) compared with the reference sequence.

As used herein, a single nucleotide variant, is phased with a nearby single nucleotide polymorphism if the former is present only on sequenced cfDNA molecules that harbor the reference (or alternate) allele at the single nucleotide polymorphism locus (i.e., they belong to the same haplotype).

Hotspot regions of somatic single nucleotide variants, as used herein, are defined as genomic regions having a statistically higher frequency of observed somatic variants than the background rate (estimated, in a preferred embodiment, from the COSMIC data set); said background rate being estimated for each gene separately. In other embodiments, other databases including but not limited to the ICGC Data Portal, Genomic Data Commons (GDC), the TP53 Database, the Cancer Hotspots and the cBioPortal.

As used herein, a "reference sequence" may be any nucleic acid sequence, a genomic sequence, the genomic sequence of an organism or subject, preferably a sequence of the human genome (e.g., hg19 or hg 38) or of a healthy individual or subject.

In the context of the present invention, a "frequency" may be used interchangeably with abundance or occurrence. In one embodiment of the invention, a variant allele frequency at a given locus describes the ratio of aligned cfDNA molecules supporting a variant allele at the given locus over the total number of aligned cfDNA molecules that span the locus.

In the context of the present invention, the term minimal residual disease (MRD) may refer to the very small number of cancer cells that remain in the body during or after treatment.

Thus, in one embodiment the method of the invention relates to and solves the problem of detecting MRD.

Herein, next-generation sequencing (NGS) may be used for nucleic acid sequence analysis, although other sequencing technologies can also be employed, which provide very accurate counting in addition to sequence information. Accordingly, other accurate counting methods, such as but not limited to digital PCR, single molecule sequencing, nanopore sequencing, DNA nanoball sequencing, sequencing by ligation, Pyrosequencing, Ion semiconductor sequencing, semiconductor sequencing, sequencing by synthesis, and microarrays can also be used instead of NGS.

As used herein, the "functional impact" of a variant determines its effect on genes, transcripts, and protein sequence, as well as regulatory regions. The functional impact could be deleterious, pathogenic, poor prognostic, disease-causing and predisposing.

As used herein, the terms "Target Capture Oligonucleotides", "TAC oligonucleotides" or "TAC" are used interchangeably and refer to short DNA oligonucleotides that are complementary to the region(s) of interest on a genomic sequence(s) of interest and which are used as bait to capture and enrich the region of interest from a large library of sequences, such as a whole genomic sequencing library prepared from a biological sample. A pool of TAC oligonucleotides is used for enrichment wherein the oligonucleotides within the pool have been optimized with regard to : (i) the length of the oligonucleotides, (ii) the distribution of the TAC oligonucleotides across the region(s) of interest, and (iii) the GC content of the TAC oligonucleotides. The number of oligonucleotides within the TAC oligonucleotide pool (pool size) has also been optimized.

### FIGURE CAPTIONS

Figure 1 illustrates the basic principles of phasing candidate somatic single nucleotide variants with germline heterozygous single nucleotide polymorphisms. This process provides a powerful method for artifact elimination.
Figure 2 is a flowchart illustrating the procedure of the two-step non-invasive targeted re-sequencing method (nested TAC oligonucleotides) of the current invention, for the determination of the presence of cell-free circulating tumor derived DNA in plasma.
Figure 3 shows the principle of TACs families.
Figure 4 shows the number of somatic variants detected in each sample. In Figure 4 the y-axis represents the number of mutations detected in each sample. The black bars correspond to Normal samples and the grey bars correspond to abnormal samples. The x-axis denotes the status of each sample (Normal or stage of cancer). The threshold to call a sample positive is represented by a horizontal solid black line. The clinical specificity of the test was 100% (11/11; 95% Cl: 72-100%) with a clinical sensitivity of 71% (10/14; 95% CI: 42-92%).

### DETAILED DESCRIPTION OF THE INVENTION

The invention relates to an in-vitro method of detecting a somatic mutation in a human,
(i) providing for a human sample, preferably a blood sample from a subject, more preferably a plasma sample, or a serum sample or a buffy coat sample,
(ii) preparing a first nucleic acid sequencing library from the nucleic acids present in said sample,
(iii) hybridizing one or more TAC oligonucleotides from a first TAC oligonucleotide pool (TAC oligonucleotide-1) to said first nucleic acid library thereby isolating a first subset of library nucleic acid molecules,
(iv) sequencing said first subset of library nucleic acid molecules and comparing the sequences determined to a human reference sequence, thereby creating a first group of putatively informative sequence variants (PISV1),
(v) hybridizing one or more TAC oligonucleotides from a second smaller TAC oligonucleotide pool comprising TAC oligonucleotides (TAC oligonucleotide-2) which are specific for the nucleic acid molecules of said first group of putatively informative sequence variants, to said first library of nucleic acid molecules thereby isolating a second subset of library nucleic acid molecules,
(vi) sequencing said second subset of library nucleic acid molecules and comparing the sequences determined to a human reference sequence, thereby creating a second group of putatively informative sequence variants (PISV2),
(vii) analyzing the putatively informative sequence variants (PISV2) thereby detecting a somatic mutation in the sample from the subject.

Steps (i) to (iv) are also referred to together as step A. Steps (v) to (vii) are also referred to together as step B.

The mere detection capability is important and that foremost in molecular research. It is important to populate databases with somatic mutations which have been identified in different cell types. Such knowledge allows for subsequent diagnosis.

The present method makes it possible to detect somatic mutations in humans using any human sample. The present method makes it possible to detect somatic mutations in humans using preferably only a blood sample. Such somatic mutation detection is useful in many diagnostic areas, such as but not limited to cancer detection. A somatic mutation is a change in the DNA sequence of a somatic cell of a multicellular organism with dedicated reproductive cells; that is, any mutation that occurs in a cell other than a gamete, germ cell, or gametocyte. Unlike germline mutations, which can be passed on to the descendants of an organism, somatic mutations are not usually transmitted to descendants. While somatic mutations are not passed down to an organism's offspring, somatic mutations will be present in all descendants of a cell within the same organism. Many cancers are the result of accumulated somatic mutations.

The term somatic generally refers to the cells of the body, in contrast to the reproductive (germline) cells, which give rise to the egg or sperm. For example, in mammals, somatic cells make up all the internal organs, skin, bones, blood and connective tissue. There are approximately 220 types of somatic cells in the human body.

Hence, in one embodiment the detection is cell type specific.

In most animals, separation of germ cells from somatic cells (germline development) occurs during early stages of development. Once this segregation has occurred in the embryo, any mutation outside of the germline cells cannot be passed down to an organism's offspring. However, somatic mutations are passed down to the progeny of a mutated cell within the same organism. A major section of an organism therefore might carry the same mutation, especially if that mutation occurs at earlier stages of development. Somatic mutations that occur later in an organism's life can be hard to detect, as they may affect only a single cell - for instance, a post-mitotic neuron; improvements in single cell sequencing are therefore an important tool for the study of somatic mutation. Both the nuclear DNA and mitochondrial DNA of a cell can accumulate mutations; somatic mitochondrial mutations have been implicated in the development of some neurodegenerative diseases. Research suggests that the frequency of mutations is generally higher in somatic cells than in cells of the germline; furthermore, there are differences in the types of mutation seen in the germ and in the soma. There is variation in mutation frequency between different somatic tissues within the same organism and between species, which is one point of application for the present invention.

Somatic mutations accumulate within an organism's cells as it ages and with each round of cell division; the role of somatic mutations in the development of cancer is well established and is implicated in the biology of aging.

Mutations in neuronal stem cells (especially during neurogenesis) and in post-mitotic neurons lead to genomic heterogeneity of neurons - referred to as "somatic brain mosaicism". The accumulation of age-related mutations in neurons may be linked to neurodegenerative diseases, including Alzheimer's disease, but the association is unproven. The majority of central-nervous system cells in adults are post-mitotic, and adult mutations might affect only a single neuron. Unlike in cancer, where mutations result in clonal proliferation, detrimental somatic mutations might contribute to neurodegenerative disease by cell death. Accurate assessment of somatic mutation burden in neurons therefore remains difficult to assess which is why the present invention is of importance.

If a mutation occurs in a somatic cell of an organism, it will be present in descendants of this cell within the same organism. The accumulation of certain mutations over generations of somatic cells is part of the process of malignant transformation, from normal cell to cancer cell.

Cells with heterozygous loss-of-function mutations (one good copy of a gene and one mutated copy) may function normally with the unmutated copy until the good copy has been spontaneously somatically mutated. This kind of mutation happens often in living organisms, but it is difficult to measure the rate. Measuring this rate is important in predicting the rate at which people may develop cancer.

Increasingly, somatic mutations are being identified in diseases other than cancer, including neurodevelopmental diseases. Somatic mutations can arise during the course of prenatal brain development and cause neurological disease-even when present at low levels of mosaicism, for example-resulting in brain malformations associated with epilepsy and intellectual disability. The present invention will allow more accurate evaluation of somatic mutations in neurodevelopmental disorders and during normal brain development.

Rare disorders that have a clear basis in somatic variations include those of the hematopoietic system, in which stems cells can mutate and expand to produce disease phenotypes. These include paroxysmal nocturnal hemoglobinuria 1 (PNH1) caused by PIG-A mutations and X-linked alpha-thalassemia mental retardation caused by mutations in ATRX. PNH1 is an acquired hemolytic anemia that presents with hemoglobinuria, abdominal pain, smooth muscle dystonias, fatigue, and thrombosis. It is caused by expansion of hematopoietic stem cells with a mutation in the PIG-A gene-a change that is acquired somatically. X-linked alpha-thalassemia mental retardation is sometimes associated with myelodysplastic syndrome, with cases often associated with somatic mutations. Interestingly, in the case of ATRX mutations, somatic variants appear to confer more severe myelodysplastic syndrome disease than do germline mutations. Clearly, the ability to clonally expand hematopoietic stem cells can provide a mechanism by which somatic mutation can confer disease risk.

Neurofibromatosis 1 (NF1), a disorder that maps to a segment of chromosome 17q, presents with cafe-au-lait spots, Lisch nodules in the eye, and fibromatous tumors of the skin. Several studies have shown that a large minority of NF1 cases are due to somatic mutations, often deletions or microdeletions in this chromosomal region (up to 40 % of cases). Other cases are caused by somatic mitochondrial DNA (mtDNA) mutations. In either case, it is clear that somatic changes are often causative of NF1. Similarly, NF2 has been shown to often be caused by somatic mutation as well (25-30 % of cases).

Diseases of other tissues can be shown to be somatic in origin by careful characterization of resected tissue. Examples include diseases of the heart and kidney. For example, mutations in connexin 40, a cardiac myocyte-expressed protein encoded by GJA5, have been shown to affect electrical communication and associate with a large minority of atrial fibrillation cases. Most of the GJA5 mutations found in cardiac myocytes of patients were not present in blood, indicating a somatic origin. A similar situation has been found in some Alport syndrome cases. Alport syndrome is an X-linked dominant disorder characterized by kidney disease, hearing loss, and eye abnormalities. It is caused by mutations in collagen IV components, mostly COL4A5. Although most Alport syndrome cases are inherited through the germline, it has been reported that males with a less severe phenotype have COL4A5 somatic mutations. As with many X-linked diseases that would otherwise be extremely severe in presentation or lethal in males, somatic mutations can present with milder forms of disease.

Somatic mutation has also played a role in some neurological diseases, including epilepsy, autism spectrum disorders (e.g., Rett syndrome), and intellectual disability, although comparisons of monozygotic twins for multiple sclerosis (MS) have been essentially negative. The latter example is based on whole genomic data of discordant monozygotic twins, but the data were derived from lymphocytes-clearly not the ideal tissue for MS. Neurological disease may be particularly sensitive to somatic mutation because even less than 10% of cells carrying a mutation can affect phenotypes based on the distribution of these cells in the brain. For example, hemimegalencephaly (HMG), which presents with enlargement and malformation of an entire hemisphere, is associated with somatic mutations of AKT3 and other mutations in the PI3K-AKT3-mTOR pathway, even when as few as 8% (and generally fewer than 35%) of cells carry the somatic mutation. However, because of the broad distribution of the mutation-carrying cells, individuals can still present with HMG. The effects of even rare somatic mutations may be due to the unique development pattern of the brain and its complex clonal migration patterns, such that clonality is not limited to adjacent or nearby cells.

Lissencephaly, or smooth brain, can be caused by mutations in two genes: Doublecortin X (DCX) or Lissencaphaly 1 (LIS1). Mutations in LIS1, which maps to 17p1, are usually lethal in males, but milder forms have been associated with somatic mosaics in two patients with predominantly posterior subcortical band heterotopia [40]. In these patients, 18-24% of blood cells and 21-34% of hair roots were mutated. Somatic mutations of DCX1 have also been shown to associate with similar disease phenotypes. As with the neurological diseases above, not all neuronal cells carry the mutations, but they do exist in leukocytes, suggesting early somatic mutation.

Mutations in the X-linked pyruvate dehydrogenase A1 (PDHA1) can present with metabolic or neurological traits. Metabolic disease usually leads to death in infancy from lactic acidosis, but the neurological form presents with symptoms including epilepsy, mental retardation, and spasticity. A continuum exists between these two presentations. A high proportion of heterozygous females present with severe disease, but a report showed that a female with mild disease had evidence of preferential X-inactivation and somatic mutation [42]. Similarly, a male with a mild form of disease had an exon skipping mutation in both skin and muscle tissue, but not lymphocytes [43]. Although limited to single clinical cases, both of these examples show that somatic mutations in a single gene can affect disease risk. And of note, both cases caused by somatic variation presented with milder forms of disease.

Lastly, autoimmune diseases can be caused by somatic mutations. A recent study of autoimmune lymphoproliferative syndrome (ALPS), a disease of benign lymphoproliferation, elevated immunoglobulins, plasma IL-10 and FAS-L, and accumulation of double-negative T cells, showed that in several cases this was due to somatic mutation (Human Somatic Variation: It's Not Just for Cancer Anymore, Chun Li & Scott M. Williams).In a preferred embodiment of the invention the invention relates to an in-vitro method of diagnosis, prognosis, treatment response control, response prediction, of a particular human disease comprising the steps of:
(i) providing for a human sample, preferably a blood sample from a subject, more preferably a plasma sample, a serum sample or a buffy coat sample,
(ii) preparing a first nucleic acid sequencing library from the nucleic acids present in said sample,
(iii) hybridizing one or more TAC oligonucleotides from a first TAC oligonucleotide pool (TAC oligonucleotide-1) to said first nucleic acid library thereby isolating a first subset of library nucleic acid molecules,
(iv) sequencing said first subset of library nucleic acid molecules and comparing the sequences determined to a human reference sequence, thereby creating a first group of putatively informative sequence variants (PISV1),
(v) hybridizing one or more TAC oligonucleotides from a second smaller TAC oligonucleotide pool comprising TAC oligonucleotides (TAC oligonucleotide-2) which are specific for the nucleic acid molecules of said first group of putatively informative sequence variants, to said first library of nucleic acid molecules thereby isolating a second subset of library nucleic acid molecules,
(vi) sequencing said second subset of library nucleic acid molecules and comparing the sequences determined to a human reference sequence, thereby creating a second group of putatively informative sequence variants (PISV2),
(vii) analyzing the putatively informative sequence variants (PISV2) thereby diagnosing, prognosing, controlling the response to a drug, for a particular human disease state.

Steps (i) to (iv) are also referred to together as step A. Steps (v) to (vii) are also referred to together as step B.

Detection and diagnosis are not the same. Diagnosis goes much further in that the quality of the mutation comes into play.

The inventors have coined the phrase nested TAC oligonucleotide enrichment method for the present very general invention. It is different from what is disclosed in a previous invention made by the same inventors: WO 2016/189388.

When providing for a human blood sample from a subject, preferably a plasma sample, a serum sample or a buffy coat sample ideally the method is performed initially on plasma and/or serum and then again on buffy coat.

Following isolation, the cell free DNA of the sample is used for sequencing library construction to make the sample compatible with a downstream sequencing technology, such as next-generation sequencing (NGS). Typically, this involves ligation of adapters onto the ends of the cell free DNA fragments, followed by amplification. Sequencing library preparation kits are commercially available.

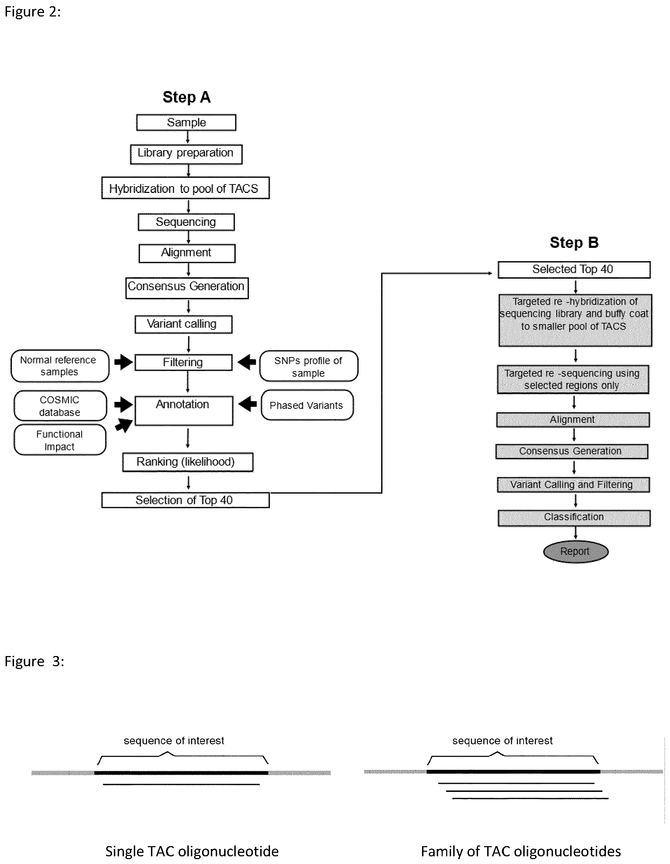

In-solution hybridization enrichment has been used in the past to enrich specific regions of interest prior to sequencing (see WO 2016/189388). However, for the methods of the invention, the target oligonucleotides (referred to as TArget Capture oligonucleotides, or TAC oligonucleotides) used to enrich for specific regions of interest have been optimized for maximum efficiency, specificity and accuracy and, furthermore, are used in families of TAC oligonucleotides, comprising a plurality of members that bind to the same genomic sequence but with differing start and/or stop positions, such that enrichment of the genomic sequences of interest is significantly improved compared to use of a single TAC oligonucleotide binding to the genomic sequence. The configuration of such families of TAC oligonucleotides is illustrated schematically in Figure 3, showing that the different start and/or stop positions of the members of the TAC oligonucleotide family when bound to the genomic sequence of interest results in a staggered binding pattern for the family members.

The use of families of TAC oligonucleotides with the TAC oligonucleotide pool that bind to each target sequence of interest, as compared to use of a single TAC oligonucleotide within the TAC oligonucleotide pool that binds to each target sequence of interest, significantly increases enrichment for the target sequences of interest, as evidenced by a greater than 50% average increase in read-depth for the family of TAC oligonucleotides versus a single TAC oligonucleotide.

Each TAC oligonucleotide family comprises a plurality of members that bind to the same genomic sequence of interest but having different start and/or stop positions with respect to a reference coordinate system for the genomic sequence of interest. Typically, the reference coordinate system that is used for analyzing human genomic DNA is the human reference genome build hg19, which is publicly available in the art, although other versions (e.g., hg38) may be used. Alternatively, the reference coordinate system can be an artificially created genome based on build hg19 that contains only the genomic sequences of interest.

Each TAC oligonucleotide family comprises at least 2 members that bind to the same genomic sequence of interest. In various embodiments, each TAC oligonucleotide family comprises at least 2 member sequences, or at least 3 member sequences, or at least 4 member sequences, or at least 5 member sequences, or at least 6 member sequences, or at least 7 member sequences, or at least 8 member sequences, or at least 9 member sequences, or at least 10 member sequences. In various embodiments, each TAC oligonucleotide family comprises 2 member sequences, or 3 member sequences, or 4 member sequences, or 5 member sequences, or 6 member sequences, or 7 member sequences, or 8 member sequences, or 9 member sequences, or 10 member sequences. In various embodiments, the plurality of TAC oligonucleotide families comprise different families having different numbers of member sequences. For example, a pool of TAC oligonucleotides can comprise one TAC oligonucleotide family that comprises 3 member sequences, another TAC oligonucleotide family that comprises 4 member sequences, and yet another TAC oligonucleotide family that comprises 5 member sequences, and the like. In one embodiment, a TAC oligonucleotide family comprises 3-5 member sequences. In another embodiment, the TAC oligonucleotide family comprises 4 member sequences.

The pool of TAC oligonucleotides comprises a plurality of TAC oligonucleotide families. Thus, a pool of TAC oligonucleotides comprises at least 2 TAC oligonucleotide families. In various embodiments, a pool of TAC oligonucleotides comprises at least 3 different TAC oligonucleotide families, or at least 5 different TAC oligonucleotide families, or at least 10 different TAC oligonucleotide families, or at least 50 different TAC oligonucleotide families, or at least 100 different TAC oligonucleotide families, or at least 500 different TAC oligonucleotide families, or at least 1000 different TAC oligonucleotide families, or at least 2000 TAC oligonucleotide families, or at least 4000 TAC oligonucleotide families, or at least 5000 TAC oligonucleotide families.

Each member within a family of TAC oligonucleotides binds to the same genomic region of interest but with different start and/or stop positions, with respect to a reference coordinate system for the genomic sequence of interest, such that the binding pattern of the members of the TAC oligonucleotide family is staggered (Figure 3). In various embodiments, the start and/or stop positions are staggered by at least 3 base pairs, or at least 4 base pairs, or at least 5 base pairs, or at least 6 base pairs, or at least 7 base pairs, or at least 8 base pairs, or at least 9 base pairs, or at least 10 base pairs, or at least 15 base pairs, or at least 20 base pairs, or at least 25 base pairs. Typically, the start and/or stop positions are staggered by 5-10 base pairs. In one embodiment, the start and/or stop positions are staggered by 5 base pairs. In another embodiment, the start and/or stop positions are staggered by 10 base pairs.

The nested TAC oligonucleotide-enrichment based method of the disclosure can be used in the detection of a wide variety of genetic abnormalities. In one embodiment, the genetic abnormality is a chromosomal aneuploidy (such as a trisomy, a partial trisomy or a monosomy). In other embodiments, the genomic abnormality is a structural abnormality, including but not limited to copy number changes including microdeletions and microduplications, insertions, translocations, inversions and small-size mutations including point mutations and mutational signatures. In another embodiment, the genetic abnormality is a chromosomal mosaicism.

In the method according to the present invention the second TAC oligonucleotide pool (TAC oligonucleotide-2) is ideally made up of a subset of the first TAC oligonucleotide pool. Alternatively, the second TAC oligonucleotide pool is newly synthesized and is preferably patient or disease specific. Fundamentally and most often, the first group of putatively informative sequence variants (PISV1) will decide which second TAC oligonucleotide pool to use. The PISV1 lay the groundwork for the diagnostic analysis and determine which loci need to be analyzed in more detail or in more depth.

In one embodiment, the TAC oligonucleotide-2 utilized are selected from a larger initial pool of TAC oligonucleotides utilized in TAC oligonucleotide-1 of the present invention, depending on the profile of each patient, i.e., the results of the first step of the analysis of a plasma sample as described in detail below in Examples 1 and 2. In various embodiments, the smaller pool of TAC oligonucleotide-2 makes a total percentage of 0.1%, 0.25%, 0.5%, 0.75 or 1.0% of the larger initial pool of TAC oligonucleotide-1.

Said first subset of library nucleic acid molecules is amplified and sequenced to obtain at least 10,000 or 20,000 or 30,000 or 50,000 cfDNA nucleic acid molecules per targeted region. Said second subset of library nucleic acid molecules is amplified and sequenced to obtain at least 100,000 or 200,000 or 300,000 or 500,000 cfDNA nucleic acid molecules per targeted region.

The filtering statistics comprise mapping quality scores and variant allele frequency thresholds computed from a distribution of erroneous variant allele frequencies estimated for each possible substitution in regions spanned by the said pool of TAC oligonucleotides using a set of normal reference samples not previously diagnosed with cancer, said erroneous variants are caused primarily by PCR or sequencing reactions.

The benefit of the TAC oligonucleotides amongst many others is the fact that less sequence artifacts are created. Newly synthesized DNA strands may however harbor sequence changes that are artifactual in cause. In the method according to the invention creating a first group of putatively informative sequence variants (PISV1) comprises the step of distinguishing between sequence variants that are a consequence of experimental artifacts and sequence variants that are present in the sample.

In the method according to the invention each sequence in said first group of putatively informative sequence variants (PISV1) is ranked according to information value based on likelihood statistics.

In the present method the likelihood statistic of a single nucleotide variant represents the probability of the single nucleotide variant to be a true somatic single nucleotide variant, wherein said likelihood statistic is computed using a regression model, wherein said regression model comprises one or more of the following steps:
(i) average mapping quality of aligned cfDNA nucleic acid molecules;
(ii) ratio of good quality to poor quality aligned cfDNA nucleic acid molecules that support the variant nucleotide;
(iii) average distance of the single nucleotide variant position from the end points of the aligned one or more cfDNA nucleic acid molecules;
(iv) Levenshtein distance of the sequence of the one or more aligned cfDNA nucleic acid molecules from the reference genome sequence;
(v) frequency of the one or more single nucleotide variant positions in a cohort of normal reference samples;
(vi) proportion of sequenced cfDNA nucleic acid molecules supporting a phased single nucleotide variant with a nearby single nucleotide polymorphism, if any, wherein the single nucleotide variant and single nucleotide polymorphism are separated by at most 110 bp;
(vii) frequency of single nucleotide variant in a disease database for example a cancer somatic mutation database;
(viii) predicted functional impact of said single nucleotide variant.

Hence, the likelihood statistic of a single nucleotide variant is computed from a regression model, comprising the following covariates: (a) average mapping quality of aligned cfDNA nucleic acid molecules; (b) ratio of good quality to poor quality aligned cfDNA nucleic acid molecules that support the variant nucleotide; (c) average distance of the single nucleotide variant position from the end points of the aligned one or more cfDNA nucleic acid molecules; (d) Levenshtein distance of the sequence of the one or more aligned cfDNA nucleic acid molecules from the reference genome sequence; (e) frequency of the one or more single nucleotide variant positions in a cohort of normal reference samples; (f) proportion of sequenced cfDNA nucleic acid molecules supporting a phased single nucleotide variant with a nearby single nucleotide polymorphism, if any, wherein the single nucleotide variant and single nucleotide polymorphism are separated by at most 110 bp; (g) frequency of single nucleotide variant in the Catalogue of Somatic Mutations in Cancer (COSMIC); and (h) the functional impact of the candidate somatic single nucleotide variant.

The invention relates to an in-vitro method of treatment response prediction of a particular human disease comprising the steps of,
(i) providing for a human sample, preferably a blood sample from a subject, more preferably a plasma sample, a serum sample or a buffy coat sample,
(ii) preparing a first nucleic acid sequencing library from the nucleic acids present in said sample,
(iii) hybridizing one or more TAC oligonucleotides from a first TAC oligonucleotide pool (TAC oligonucleotide-1) to said first nucleic acid library thereby isolating a first subset of library nucleic acid molecules,
(iv) sequencing said first subset of library nucleic acid molecules and comparing the sequences determined to a human reference sequence, thereby creating a first group of putatively informative sequence variants (PISV1),
(v) hybridizing one or more TAC oligonucleotides from a second smaller TAC oligonucleotide pool comprising TAC oligonucleotides (TAC oligonucleotide-2) which are specific for the nucleic acid molecules of said first group of putatively informative sequence variants, to said first library of nucleic acid molecules thereby isolating a second subset of library nucleic acid molecules,
(vi) sequencing said second subset of library nucleic acid molecules and comparing the sequences determined to a human reference sequence, thereby creating a second group of putatively informative sequence variants (PISV2),
(vii) analyzing the putatively informative sequence variants (PISV2) thereby predicting response to a drug, for a particular human disease state.

Steps (i) to (iv) are also referred to together as step A. Steps (v) to (vii) are also referred to together as step B.

A cancer is a complex disease. Cancer tissues are most often heterogenic when it comes to the composition of cancer mutations in the tissues. Each cell might harbor different mutations as outlined above. A selected drug will be responder specific but only for selected group of mutations and selected metabolic pathway. The cancer tissues might only harbor 20% of mutation "A" but 80% of a different cancer mutation "B". By using the method of the present invention it is possible to mirror the heterogeneity and rank mutations in their response likelihood to various drugs acting on different pathways but also in their quantitative presence and driving cancer force.

In a preferred embodiment the method relates to a method prognosis of a particular human disease comprising the steps of:
(i) providing for a human sample, preferably a blood sample from a subject, more preferably a plasma sample, a serum sample or a buffy coat sample;
(ii) preparing a first nucleic acid sequencing library from the nucleic acids present in said sample;
(iii) hybridizing one or more TAC oligonucleotides from a first TAC oligonucleotide pool (TAC oligonucleotide-1) to said first nucleic acid library thereby isolating a first subset of library nucleic acid molecules;
(iv) sequencing said first subset of library nucleic acid molecules and comparing the sequences determined to a human reference sequence, thereby creating a first group of putatively informative sequence variants (PISV1);
(v) hybridizing one or more TAC oligonucleotides from a second smaller TAC oligonucleotide pool comprising TAC oligonucleotides (TAC oligonucleotide-2) which are specific for the nucleic acid molecules of said first group of putatively informative sequence variants, to said first library of nucleic acid molecules thereby isolating a second subset of library nucleic acid molecules;
(vi) sequencing said second subset of library nucleic acid molecules and comparing the sequences determined to a human reference sequence, thereby creating a second group of putatively informative sequence variants (PISV2),
(v) analyzing the putatively informative sequence variants (PISV2) thereby prognosing a particular human disease state.

Prognosis is a medical term for predicting the likely or expected development of a disease, including whether the signs and symptoms will improve or worsen, and how quickly, or remain stable over time. When applied to large statistical populations, prognostic estimates can be very accurate: for example, the statement "45% of patients with severe septic shock will die within 28 days" can be made with some confidence, because previous research found that this proportion of patients died.

This statistical information does not apply to the prognosis for each individual patient, because patient-specific factors can substantially change the expected course of the disease: additional information is needed to determine whether a patient belongs to the 45% who will die, or to the 55% who will survive. By using the method of the present invention and setting up a sample testing regimen prognosis can be accomplished. For example, a fluid and dynamic cancer mutation model is established, weighing each mutation differentially and thereby prognosing disease progression and/or disease outcome.

The invention relates to an in-vitro method of treatment response control of a particular human disease comprising the steps of,
(i) providing for a human sample, preferably a blood sample from a subject, more preferably a plasma sample, a serum sample or a buffy coat sample,
(ii) preparing a first nucleic acid sequencing library from the nucleic acids present in said sample,
(iii) hybridizing one or more TAC oligonucleotides from a first TAC oligonucleotide pool (TAC oligonucleotide-1) to said first nucleic acid library thereby isolating a first subset of library nucleic acid molecules,
(iv) sequencing said first subset of library nucleic acid molecules and comparing the sequences determined to a human reference sequence, thereby creating a first group of putatively informative sequence variants (PISV1),
(v) hybridizing one or more TAC oligonucleotides from a second smaller TAC oligonucleotide pool comprising TAC oligonucleotides (TAC oligonucleotide-2) which are specific for the nucleic acid molecules of said first group of putatively informative sequence variants, to said first library of nucleic acid molecules thereby isolating a second subset of library nucleic acid molecules,
(vi) sequencing said second subset of library nucleic acid molecules and comparing the sequences determined to a human reference sequence, thereby creating a second group of putatively informative sequence variants (PISV2),
(vii) analyzing the putatively informative sequence variants (PISV2) thereby controlling the response to a drug, for a particular human disease state.

Steps (i) to (iv) are also referred to together as step A. Steps (v) to (vii) are also referred to together as step B.

Therapeutic drug monitoring (TDM) is a branch of clinical chemistry and clinical pharmacology that specializes in the measurement of medication levels in blood. Its main focus is on drugs with a narrow therapeutic range, i.e., drugs that can easily be under- or overdosed. TDM aimed at improving patient care by individually adjusting the dose of drugs for which clinical experience or clinical trials have shown it improved outcome in the general or special populations.

In cancers this is more complex. Cancer tissues are most often heterogenic when it comes to the composition of cancer mutations in the tissues. Each cell might harbor different mutations thereby making the tissues a complex heterogenic mutation 3D mutation mode. By using the method of the present invention and setting up a sample testing regimen monitoring/control can be accomplished. For example, a fluid and dynamic cancer mutation model is established, weighing each mutation differentially and thereby prognosing disease progression and/or disease outcome. The selected drug will be responder specific but only for selected group of mutations and selected metabolic pathway. The present method will monitor pressure put on that pathway and quantitative mutation outcome and certain time points before, during and after treatment.

In the method of the invention ideally the single nucleotide variant and single nucleotide polymorphism are separated by at most by 100 bp, or 120 bp or 130 bp, or 140 bp, or 150 bp.

In a preferred embodiment, preparing the DNA sequencing library comprises the step of including unique molecular identifiers (UMIs) to uniquely tag each molecule and to create UMI families.

Preferably, in the method the sample is a plasma sample, a serum sample, or a buffy coat sample and the nucleic acid in the sample is cell-free DNA (cfDNA).

Preferably, putatively informative nucleic acid sequence variants are somatic DNA variants and are selected from the group comprising frameshift mutations, insertion deletion mutations and, single nucleotide exchanges (single nucleotide mutations). Most preferably the mutations analyzed are single nucleotide exchanges (single nucleotide mutations).

In other embodiments, the method of the disclosure can be used in the detection of a wide variety of genetic abnormalities. In one embodiment, the genetic abnormality is a chromosomal aneuploidy (such as a trisomy, a partial trisomy or a monosomy) . In yet other embodiments, the genomic abnormality is a structural abnormality, including but not limited to copy number changes including microdeletions and microduplications, insertions, translocations, inversions and small-size mutations including point mutations and mutational signatures. In another embodiment, the genetic abnormality is a chromosomal mosaicism.

Preferably, in the method according to the invention the first group of putatively informative sequence variants (PISV1) comprises between 5 and 200 PISVs, 8 and 100 PISVs, 10 and 80 PISVs and, 20 and 60 PISVs, preferably about 40 PISVs.

Ideally, in the method of the invention the diagnosis is the diagnosis of a disease selected from the group comprising a chronic disease, a congenital disease, a genetic disease, a hereditary disease, an acute disease and an idiopathic disease.

In the method according to the invention the first TAC oligonucleotide pool comprises oligonucleotides that are specific for known genetic disease loci of the disease to be diagnosed.

In the method according to the invention the disease is preferably selected from the group comprising cancer, neurodegenerative diseases, McCune-Albright Syndrome, hematological and immune-related disorders, paroxysmal nocturnal hemoglobinuria 1, X-linked alpha-thalassemia mental retardation, Alport-syndrome, genetic diseases, autoimmune diseases, kidney diseases, cardiovascular diseases, mental diseases, aging diseases, neuromuscular diseases, reproductive diseases, pulmonary diseases, organ transplantation monitoring and sepsis.

In the method according to the invention the cancer is selected from carcinoma, sarcoma, melanoma, lymphoma, adenoma and leukemia.

In various embodiments of the method, the top 20, 30, 40, 50, 60, 70, 80, 90 single variant positions are selected from the plurality of ranked single nucleotide variant positions of the first group of putatively informative sequence variants (PISV1).

In different embodiments of the method, the presence of cell-free tumor DNA nucleic acid molecules is determined if more than 1%, or 10%, or 15% or 20%, or 25% or 1 mutation, or 2 or 3 or 4 mutations from the plurality of single nucleotide variants selected in Step A are detected in Step B.

In different embodiments, in addition to a plurality of single nucleotide variant positions, the method can also identify a plurality of small insertions and deletions.

The method of this disclosure can be used to detect relapse and to monitor MRD, wherein detection of at least a fraction of ctDNA remaining in a patient after treatment, can indicate inadequate treatment or tumor recurrence.

The method for detecting cell free tumor DNA can be additionally used in a variety of different clinical circumstances in the oncology field. For example, the method can be used for making an initial cancer diagnosis in a subject suspected of having cancer. Accordingly in one embodiment, the method further comprises making a diagnosis of the subject based on detection of at least a fraction of cell-free tumor DNA in a sample obtained from said subject.

The method can be further used to select an appropriate treatment regimen for a patient diagnosed with cancer, wherein the treatment regimen is designed to be effective against a tumor having the tumor biomarkers detected in the patient's tumor (i.e., known in the art as personalized medicine). Accordingly, in another embodiment, the method further comprises selecting a therapeutic regimen for the subject based on detection of at least one tumor biomarker sequence.

In one aspect, the method can be used to monitor the efficacy of a therapeutic regiment, wherein changes in tumor biomarker detection are used as an indicator of treatment efficacy. Accordingly, in another embodiment, the method further comprises monitoring treatment efficacy of a therapeutic regimen in the subject based on detection of at least one tumor biomarker sequence.

In a different embodiment, the method can be used to detect cancer-related germline (hereditary) mutations in patients with cancer or individuals suspected of a cancer pre-disposing syndrome wherein detection of at least one germline mutation is used as an indicator for having a cancer predisposing syndrome. Accordingly, in another embodiment, the method further comprises diagnosing a patient or an individual with a hereditary cancer pre-disposing syndrome thus allowing for early medical intervention, treatment selection and close monitoring.

In a different embodiment the method can be used in detecting donor-derived cell-free DNA in transplantation, as a potential rejection biomarker. Donor-derived cell-free DNA (dd-cfDNA) is cfDNA that is exogenous to the patient and comes from a transplanted organ. The increase in dd-cfDNA concentration occurs even before the creatinine level starts rising, which may enable early diagnosis of transplant injury and adequate treatment to avoid premature graft loss (Martuszewski A, et al, (2021), J Clin Med, 10(2):192).

In one embodiment of the invention the invention relates to an in-vitro method of for the detection of donor-derived cell-free DNA after transplantation, the method comprising the steps of:
(i) providing for a human sample, preferably a blood sample from a subject, more preferably a plasma sample, a serum sample or a buffy coat sample,
(ii) preparing a first nucleic acid sequencing library from the nucleic acids present in said sample,
(iii) hybridizing one or more TAC oligonucleotides from a first TAC oligonucleotide pool (TAC oligonucleotide-1) to said first nucleic acid library thereby isolating a first subset of library nucleic acid molecules,
(iv) sequencing said first subset of library nucleic acid molecules and comparing the sequences determined to a human reference sequence, thereby creating a first group of putatively informative sequence variants (PISV1),
(v) hybridizing one or more TAC oligonucleotides from a second smaller TAC oligonucleotide pool comprising TAC oligonucleotides (TAC oligonucleotide-2) which are specific for the nucleic acid molecules of said first group of putatively informative sequence variants, to said first library of nucleic acid molecules thereby isolating a second subset of library nucleic acid molecules,
(vi) sequencing said second subset of library nucleic acid molecules and comparing the sequences determined to a human reference sequence, thereby creating a second group of putatively informative sequence variants (PISV2),
(vii) analyzing the putatively informative sequence variants (PISV2) thereby diagnosing, prognosing, controlling the response to a drug, for a particular human disease state.

The method disclosed herein can also be used in detecting sepsis in cell-free DNA in patients with suspected bloodstream infection. Plasma microbial cell-free DNA biomarkers can enable the identification of patients with sepsis. Cell-free DNA can be released from cells undergoing necrosis or apoptosis as well as from pathogens. The detection of sepsis via cell-free DNA assessment can improve and accelerate diagnosis and prompt medical intervention in the ICU.

In one embodiment, a DNA sequencing library is prepared from a subject's sample having or suspected of having tumor or has had tumor resection. Said sequencing library is subsequently hybridized to a pool of Target Capture Oligonucleotides (TAC oligonucleotides) targeting regions of interest wherein said regions of interest are regions which contain mutations known to be associated with cancer.

In one embodiment, following preparation of the sequencing library, the region(s) of interest is enriched by (a) hybridizing the pool of TAC oligonucleotides to the sequencing library and (b) by isolating sequences within the sequencing library that bind to the TAC oligonucleotides. To facilitate isolation of the desired enriched sequences, typically the TAC oligonucleotides are modified in such a way that sequences that hybridize to the TAC oligonucleotides can be separated from sequences that do not hybridize to the TAC oligonucleotides. In one embodiment, this is achieved by fixing the TAC oligonucleotides to a solid support, allowing for physical separation of sequences that bind the TAC oligonucleotides from sequences which do not bind the TAC oligonucleotides. For example, each sequence within the pool of TAC oligonucleotides can be labelled with biotin and the pool can then be bound to beads coated with a biotin-binding substance, such as streptavidin or avidin. In a preferred embodiment, the TAC oligonucleotides are labelled with biotin and are bound to streptavidin-coated magnetic beads. The person skilled in the art will however appreciate that other affinity binding systems are known in the art and can be used instead of the biotinstreptavidin/avidin method. For example, an antibody-based system can be used, in which the TAC oligonucleotides are labelled with an antigen and are then bound to antibody-coated beads. Moreover, the TAC oligonucleotides can incorporate on one end a sequence tag and can be bound to a solid support via a complementary sequence on the solid support that hybridizes to the sequence tag. Furthermore, in addition to the magnetic beads, other types of solid support can be used, such as polymer beads.

Following enrichment of the regions of interest using the TAC oligonucleotides, thereby forming an enriched library, in one embodiment, the members of the enriched library are eluted from the solid support and are amplified and sequenced using standard methods known in the art. Standard Illumina NGS is typically used, which provides very accurate counting in addition to sequence information, although other sequencing technologies can also be employed. The enriched library is amplified and sequenced to obtain, in one embodiment, at least 10,000 cfDNA nucleic acid molecules per targeted region. In other embodiments of the method, a sequencing DNA library is also prepared for the corresponding buffy coat, hybridized with the selected pool of TAC oligonucleotides and sequenced to obtain at least 10,000 cfDNA nucleic acid molecules per targeted region.

In one embodiment, the sequencing data obtained from the sequencing step is processed, by a computer system, to align at least a portion of the sequenced cfDNA nucleic acid molecules on the hg19 human reference genome assembly. Following alignment, a consensus sequence construction for each UMI family is generated (http://fulcrumgenomics.github.io/fgbio/). In another embodiment the alignment in step B is performed using the consensus sequence specific to the patient generated from A.

Variant calling is subsequently performed in an ultra-sensitive manner by identifying a plurality of single nucleotide variant positions relative to the reference genome sequence, wherein, in one embodiment of the current invention, at least one, and at most 25%, of the sequenced cfDNA nucleic acid molecules that span the one or more single nucleotide variant positions, support a variant nucleotide. The term "variant nucleotide" as used herein, generally refers to a single base pair mismatch compared with the reference sequence. In other embodiments of the current method, a plurality of single nucleotide variant positions relative to the reference genome sequence is identified, wherein, at least one, and at most 10% or 20% or 30% or 40% of the sequenced cfDNA nucleic acid molecules that span the one or more single nucleotide variant positions, support a variant nucleotide. A subsequent filtering is performed using mapping and sequencing quality scores and thresholds, computed from a distribution of erroneous allele frequencies estimated for each possible substitution spanned by said pool of TAC oligonucleotides, using a set of normal reference sample not previously diagnosed with cancer. In different embodiments, in addition to a plurality of single nucleotide variant positions, the method can also identify a plurality of small insertions and deletions.

Following the filtering step, a likelihood statistic is then computed for each candidate somatic single nucleotide variant position using a regression model. In one embodiment, the regression model is a logistic regression model, and the likelihood statistic is the log odds ratio of the probability of a candidate to be a true somatic variant over the probability of a candidate to be an artifact. In one embodiment, the regression model is trained on at least 7,000,000 aligned cfDNA nucleic acid molecules from 71 normal reference samples not previously diagnosed with cancer, and 45 cancer patients (Non-Small Cell Lung Cancer, NSCLC) with at least three known somatic variants previously characterized using at least one tissue specimen. Said regression model is comprising at least one of the following covariates:
R1) average mapping quality of aligned cfDNA nucleic acid molecules;
R2) ratio of good quality to poor quality aligned cfDNA nucleic acid molecules that support the variant nucleotide;
R3) average distance of the single nucleotide variant position from the end points of the aligned one or more cfDNA nucleic acid molecules;
R4) Levenshtein distance of the sequence of the one or more aligned cfDNA nucleic acid molecules from the reference genome sequence;
R5) frequency of the one or more single nucleotide variant positions in a cohort of normal reference samples;
R6) proportion of sequenced cfDNA nucleic acid molecules supporting a phased single nucleotide variant with a nearby single nucleotide polymorphism, if any, wherein the single nucleotide variant and single nucleotide polymorphism are separated by at most 110 bp;
R7) frequency of single nucleotide variant in the Catalogue Of Somatic Mutations In Cancer (COSMIC);
R8) predicted functional impact.

A ranking in descending log odds likelihood order for the plurality of candidate somatic single nucleotide variant positions is followed, in one embodiment, by a selection of the top 40 candidate somatic single nucleotide variant positions for subsequent analysis. In one aspect, a small number of single nucleotide variant positions are selected for targeted deep re-sequencing to significantly minimize cost. In a preferred embodiment, the top 40 candidate somatic single nucleotide variant positions are selected for targeted deep re-sequencing. In various other embodiments, the top 20, or 30, or 40, or 50, or 60, or 70, or 80, or 90 candidate somatic single nucleotide variant positions are selected for targeted deep re-sequencing. The selection of the top 40 single nucleotide variants, marks the completion of the first step (Step A) of the method.

The second step, (Step B), of the method utilizes the same DNA sequencing library as step A, wherein the said DNA sequencing library is re-hybridized to a smaller pool of TAC oligonucleotides derived from the larger initial pool of TAC oligonucleotides of Step A, said a smaller pool of TAC oligonucleotides designed to capture genomic regions spanning the selected top 40 candidate somatic single nucleotide variant positions from Step A, to obtain an enriched library.

In a preferred embodiment of the invention the second TAC oligonucleotide pool (TAC oligonucleotide-2) comprises a defined fraction of the first TAC oligonucleotide pool (TAC oligonucleotide-1) and it makes up 0.1%, or 0.25%, or 0.5%, or 0.75% or 1.0% but most preferably about 0.5% of said second TAC oligonucleotide pool (TAC oligonucleotide-2). That means that a selected group or pool of TAC oligonucleotides is chosen from the first pool for the second smaller pool.

Following the re-hybridization step to said smaller pool of TAC oligonucleotides, the enriched library is amplified and sequenced to obtain, in one embodiment, at least 200,000 cfDNA nucleic acid sequences per targeted region.. A sequencing DNA library is also prepared for the corresponding buffy coat, hybridized with the selected pool of TAC oligonucleotides and sequenced to obtain at least 200,000 cfDNA nucleic acid sequences per targeted region. This is preformed to remove somatic variants stemming from clonal hematopoiesis. In other embodiments of the method, a sequencing DNA library is also prepared for the corresponding buffy coat, hybridized with the selected pool of TAC oligonucleotides and sequenced to obtain at least 100,000 or 200,000 or 300,000 or 400,000 or 500,000 cfDNA nucleic acid molecules per targeted region. The sequencing data are aligned on a reference genome sequence. In one embodiment the reference genome is the build hg19. In another embodiment the reference genome is the build hg38.

As people age, their tissues accumulate an increasing number of somatic mutations. While most of these mutations are of little or no functional consequence, a mutation may arise that confers a fitness advantage on a cell. When this process happens in the hematopoietic system, a substantial proportion of circulating blood cells may derive from a single mutated stem cell. This outgrowth, called "clonal hematopoiesis," is highly prevalent in the elderly population.

The sequencing data are aligned on a reference genome sequence. In one embodiment the reference genome is the build hg19. In another embodiment the reference genome is the build hg38.

Following alignment to the reference genome sequence, a classification model is used to determine whether a candidate somatic single nucleotide variant, from the plurality of the list of selected candidate somatic single nucleotide variants, is present in the sample, after the removal of any variants resulting from clonal hematopoiesis. Said classification model is an ensemble learning method. In one embodiment, said method is Decision Random Forest. In other embodiments, said method is a Supporting Vector Machine or a Naive Bayes Classifier, or a logistic regression model or a Neural Network. The classification method is trained using at least 7,000,000 aligned cfDNA molecules from both normal reference samples (not previously diagnosed with cancer) and cancer patients with known somatic variants previously characterized using at least one tissue specimen. The said classification model comprises at least one of the following covariates:
C1) number of cfDNA nucleic acid molecules in which the variant allele is present;
C2) the average mapping quality of sequenced cfDNA nucleic acid molecules, relative to the reference genome sequence;
C3) ratio of good quality to poor quality sequenced cfDNA nucleic acid molecules, that support the variant;
C4) proportion of sequenced cfDNA nucleic acid molecules supporting a phased single nucleotide variant with a nearby single nucleotide polymorphism, if any, wherein the single nucleotide variant and single nucleotide polymorphism are separated by at most 110 bp;
C5) frequency of single nucleotide variant in the Catalogue Of Somatic Mutations In Cancer (COSMIC).

In one embodiment, the output of the classifier is the estimate class/status for each candidate somatic single nucleotide variant, i.e., detected/not detected. The presence of cell-free tumor DNA nucleic acid molecules in the sample is determined by applying the following rule: if more than 5% of the plurality of the selected candidate somatic single nucleotide variants are detected in the targeted re-sequencing step of the method (Step B), then the sample is considered positive (i.e., cell-free tumor DNA nucleic acid molecules are detected in the sample), otherwise the sample is considered negative (i.e., cell-free tumor DNA nucleic acid molecules are not detected in the sample). In other embodiments of the method, the presence of cell-free tumor DNA nucleic acid molecules is determined if more than 1% or 10%, or 15% or 20% or 25% of the plurality of single nucleotide variants selected in step A are also detected in step B.

### EXAMPLES

### Method Steps

### Sample collection and preparation

For the detection of cell-free, circulating tumor DNA nucleic acid, the sample is a biological sample obtained from a subject having or suspected of having a tumor. In one embodiment, the DNA sample is obtained from a human subject. In one embodiment the sample comprises cell-free tumor DNA (cftDNA). In a preferred embodiment, the oncology sample is a patient plasma sample, prepared from patient peripheral blood. Thus, the sample can be a liquid biopsy sample that is obtained non-invasively from a patient's blood sample, thereby potentially allowing for early detection of cancer prior to development of a detectable or palpable tumor. In another embodiment the sample is a patient's serum, buffy coat, urine, sputum, ascites, cerebrospinal fluid or pleural effusion. In one embodiment, the oncology sample is a sample of tissue that has or is suspected of having cancer (e.g., tissue from a tumor biopsy). In yet another embodiment, the sample is a stool sample. In another embodiment, the oncology sample is a patient's healthy cells such as buffy coat, prepared from patient peripheral blood, or buccal swab or healthy tissue adjacent to the tumor or another source of healthy cells. Thus, the healthy cells can provide a source of DNA that allows for detection of germline mutations, and comparison with tumor DNA.

The one embodiment of the invention the buffy coat of the tested biological sample is processed following the steps described in Step B, and subsequently the candidate somatic single nucleotide variants detected in both Step A and the buffy coat are removed from the classification model to determine the presence of cell-free tumor-derived DNA nucleic acid molecules.

In one embodiment, plasma samples were obtained from human subjects having or suspected of having a tumor. For the biological sample preparation, typically plasma DNA is extracted using standard techniques known in the art, a non-limiting example of which is the Qiagen DNeasy Blood and Tissue protocol. In another embodiment, cell-free DNA is isolated from plasma using standard techniques, a non-limiting example of which is the Qiasymphony (Qiagen) protocol.

### Sequencing library preparation

Following isolation, in one embodiment, the cell-free DNA of the sample is used for sequencing library construction to make the sample compatible with a downstream sequencing technology, such as NGS. Typically, this involves ligation of adapters onto the ends of the cell-free DNA fragments, followed by amplification. Sequencing library preparation kits are commercially available. In a different embodiment, nuclear DNA (a non-limiting example of which is DNA extracted from tissue of buffy coat) is fragmented using standard techniques, including but not limited to sonication technique. Fragmented nuclear DNA is then subjected to the same downstream procedures for cell-free DNA, described in this paragraph.

Extracted DNA from plasma samples was used for sequencing library construction. Standard library preparation methods were used with the following modifications: a negative control extraction library was prepared separately to monitor any contamination introduced during the procedure. During this step, 5' and 3' overhangs were filled-in, by adding 12 units of T4 polymerase (NEB), 5 units of Taq polymerase were used to add adenine to the 3' end of each fragment, while 5' phosphates were attached using 40 units of T4 polynucleotide kinase (NEB) in a 10 µl reaction and subsequent incubation at 20°C for 30 minutes and then 65° C for 30 minutes.

Subsequently, adaptors P5 and P7 with duplex unique molecular identifiers, (UMIs), (IDT CS adapters) were ligated at 1:5 dilution to both ends of the DNA using 5 units of T4 DNA ligase (NEB) in a 40 µl reaction for 15 minutes at room temperature, followed by purification using Ampure beads with ratio 1.0x. Library amplification was performed using a Fusion polymerase (Herculase II Fusion DNA polymerase (Agilent Technologies) or Pfusion High Fidelity Polymerase (NEB)) in 50 µl reactions and with the following cycling conditions, 98 °C for 3 minutes; followed by 11 cycles at 98 °C for 30 seconds, 60°C for 30 seconds, 72°C for 30 seconds and finally 72°C for 3 minutes (modified from Koumbaris, G. et al. (2016) Clinical Chemistry, 62(6), pp.848-855). The final library products were purified using Ampure beads with ratio 1.5x.

### TArget Capture Oligonucleotide (TAC oligonucleotides) Design and Preparation

This example describes preparation of custom TAC oligonucleotides for the detection of cell-free tumor-derived DNA in plasma. The genomic target-loci used for TAC oligonucleotide design were selected based on their GC content and their distance from repetitive elements (minimum 50 bp away). TAC oligonucleotide size can be variable.

In a preferred embodiment, each sequence within the pool of TAC oligonucleotides is between 150-260 base pairs in length. In various other embodiments, each sequence within the pool of TAC oligonucleotides is between 100-200 base pairs, 200-260 base pair, 100-350 base pairs, or 100-500 base pairs in length. The TAC oligonucleotides were prepared by simplex polymerase chain reaction using standard Taq polymerase, primers designed to amplify the target-loci, and normal DNA used as template.

All custom TAC oligonucleotides were generated using the following cycling conditions: 95°C for 3 minutes; 40 cycles at 95°C for 15 seconds, 60°C for 15 seconds, 72°C for 12 seconds; and 72°C for 12 seconds, followed by verification via agarose gel electrophoresis and purification using standard PCR clean up kits such as the Qiaquick PCR Purification Kit (Qiagen) or the NucleoSpin 96 PCR clean-up (Mackerey Nagel) or the Agencourt AMPure XP for PCR Purification (Beckman Coulter). Concentration was measured by Nanodrop (Thermo Scientific).

### TAC Oligonucleotide Biotinylation

TAC oligonucleotides were prepared for hybridization, as previously described (Koumbaris, G. et al. (2016) Clinical chemistry, 62(6), pp.848-855), starting with blunt ending with the Quick Blunting Kit (NEB) and incubation at room temperature for 30 minutes. Reaction products were subsequently purified using the MinElute kit (Qiagen) and were ligated with a biotin adaptor using the Quick Ligation Kit (NEB) in a 40 µl reaction at RT for 15 minutes. The reaction products were purified with the Min Elute kit (Qiagen) and were denatured into single stranded DNA prior to immobilization on streptavidin coated magnetic beads (Invitrogen).

### TAC Oligonucleotide Hybridization

Amplified libraries were mixed with blocking oligos (Koumbaris, G. et al. (2016) Clinical Chemistry, 62(6), pp.848-855) (200 1-1M), 50 µg of Cot-1 DNA (Invitrogen), 50 µg of Salmon Sperm DNA (Invitrogen), Agilent hybridization buffer 2x, Agilent blocking agent 10X, and were heated 98°C for 3 minutes to denature the DNA strands. Denaturation was followed by a 30-minute incubation step at 37°C, to block repetitive elements and adaptor sequences. The resulting mixture was then added to the biotinylated TAC oligonucleotides. All samples were incubated in a rotating incubator for 12-48 hours at 66°C. After incubation, the beads were washed as described previously and DNA was eluted by heating (Koumbaris, G. et al. (2016) Clinical chemistry, 62(6), pp.848-855). Eluted products were amplified using outer-bound adaptor primers. Enriched amplified products were re-captured using the same bait pool, re-eluted and amplified using same above protocol. Double enriched amplified products were pooled equimolarly and sequenced on a suitable platform.

In one embodiment, a plurality of TAC oligonucleotide families used in the method binds to a plurality of regions known to be associated with cancer (hereby referred to as regions of interest). Said regions of interest, as used herein, are regions which have point mutations known to be associated with cancer. Said regions were extracted from the COSMIC database. An extensive catalogue of known cancer-associated mutations is known in the art, referred to as COSMIC (Catalogue of Somatic Mutations in Cancer), described in for example, Forbes, S.A. et al. (2016) Curr. Protocol Hum. Genetic 91:10.11.1-10.11.37; Forbes, S.A. et al. (2017) Nucl. Acids Res. 45:0777-0783; and Prior et al. (2012) Cancer Res. 72:2457-2467. The COSMIC database is publicly available at www.cancer.sanger.ac.uk. In addition to the COSMIC catalogue, other complications of tumor biomarker mutations have been described in the art, non-limiting examples of which include the ENCODE Project, which describes mutations in the regulatory sites of oncogenes (see e.g., Shar, N.A. et al. (2016) Mol. Cane. 15:76) and ClinVar, a National Center for Biotechnology Information (NCBI) database for genomic variations associated with human health. The ClinVar database is publicly available at www.ncbi.nlm.nih.gov/clinvar.

The regions of interest are enriched by hybridizing the pool of TAC oligonucleotides to the sequencing library, followed by isolation of those sequences within the sequencing library that bind to the TAC oligonucleotides. To facilitate isolation of the desired, enriched sequences, typically the TAC oligonucleotide sequences are modified in such a way that sequences that hybridize to the TAC oligonucleotides can be separated from sequences that do not hybridize to the TAC oligonucleotides. Typically, this is achieved by fixing the TAC oligonucleotides to a solid support. This allows for physical separation of those sequences that bind the TAC oligonucleotides from those sequences that do not bind the TAC oligonucleotides. For example, each sequence within the pool of TAC oligonucleotides can be labelled with biotin and the pool can then be bound to beads coated with a biotin-binding substance, such as streptavidin or avidin. In a preferred embodiment, the TAC oligonucleotides are labelled with biotin and are bound to streptavidin-coated magnetic beads.

In certain embodiments, the members of the sequencing library that bind to the pool of TAC oligonucleotides are fully complementary to the TAC oligonucleotides. In other embodiments, the members of the sequencing library that bind to the pool of TAC oligonucleotides are partially complementary to the TAC oligonucleotides. For example, in certain circumstances, it may be desirable to utilize and analyze data that are from DNA fragments which are products of the enrichment process but which do not necessarily belong to the genomic regions of interest (i.e., such DNA fragments could bind to the TAC oligonucleotides because of part homologies [partial complementarity] with the TAC oligonucleotides, and when sequenced would produce very low coverage throughout the genome in non-TAC oligonucleotide coordinates).

Following enrichment of the sequences of interest using the TAC oligonucleotides, thereby forming an enriched library, the members of the enriched library are eluted from the solid support and are amplified and sequenced using standard methods known in the art.

The pool of TAC oligonucleotides and families of TAC oligonucleotides used in the method of detecting cell-free tumor-derived DNA in plasma can include any of the designed features described herein. In various embodiments, the pool of TAC oligonucleotides comprises at least 5, 10, 50, or 100 different TAC oligonucleotide families, or more. In various embodiments each TAC oligonucleotide family comprises at least 2, at least 3, at least 4 or at least 5 different member sequences. In one embodiment, each TAC oligonucleotide family comprises 4 different member sequences. In various embodiments, the start and/or stop positions for the member sequences within a TAC oligonucleotide family, with respect to a reference coordinate system for the genomic sequence of interest, are staggered by at least 5 base pairs, or at least 10 base pairs, or by 5-10 base pairs.

### Alignment to the human genome and consensus sequences generation

In one embodiment, for each sample, the bioinformatic pipeline routine described below was applied in order to align the sample's sequenced DNA fragments to the human reference genome. Targeted paired-end read fragments obtained from NGS results were processed to remove adaptor sequences and poor quality reads (Q-score<25) using the cutadapt software (Martin, M. et al. (2011) EMB.netJournal 17.1). The quality of the raw and/or processed reads as well as any descriptive statistics which aid in the assessment of quality check of the sample's sequencing output were obtained using the FastQC software (Babraham Institute (2015) FastQC) and/or other custom-built software. Processed reads which were at least 25 bases long were processed within the FGBIO (https://bio.tools/fgbio) UMI-based bioinformatics suite to perform alignment and create UMI families (same start, stop coordinate and UMIs sequences), generate a consensus sequence per UMI family in binary alignment format. Where applicable, sequencing output pertaining to the same sample but processed on separate sequencing lanes, was merged into a single sequencing output file.

### Data Analysis

Variant calling is performed in an ultra-sensitive manner by identifying a plurality of single nucleotide variant positions relative to the reference genome sequence, wherein, at least one, and at most 25%, of the sequenced cfDNA nucleic acid molecules that span the one or more single nucleotide variant positions, support a variant (mismatch with the reference sequence) nucleotide. In other embodiments of the method, a plurality of single nucleotide variant positions relative to the reference genome sequence is identified, wherein, at least one, and at most 10% or 20% or 30% or 40% of the sequenced cfDNA nucleic acid molecules that span the one or more single nucleotide variant positions, support a variant nucleotide. A subsequent filtering is performed using mapping and sequencing quality scores and thresholds computed from a variant allele frequency distribution of erroneous variants estimated for each possible substitution spanned by the said pool of TAC oligonucleotides using a set of normal reference sample not previously diagnosed with cancer. A likelihood statistic is then computed for each candidate somatic single nucleotide variant using a regression model. In one embodiment, the regression model is a logistic regression model, and the likelihood statistic is the log odds ratio of the probability of a candidate to be a true somatic variant over the probability of a candidate to be an artifact. In one embodiment, the regression model is trained on at least 7,000,000 aligned cfDNA nucleic acid molecules from 71 normal reference samples not previously diagnosed with cancer and 45 cancer patients, with at least three known somatic variants previously characterised using at least one tissue specimen. The said regression model is comprising at least one of the following covariates:
R1) average mapping quality of aligned cfDNA nucleic acid molecules;
R2) ratio of good quality to poor quality aligned cfDNA nucleic acid molecules that support the variant nucleotide;
R3) average distance of the single nucleotide variant position from the end points of the aligned one or more cfDNA nucleic acid molecules;
R4) Levenshtein distance of the sequence of the one or more aligned cfDNA nucleic acid molecules from the reference genome sequence;
R5) frequency of the one or more single nucleotide variant positions in a cohort of normal reference samples;
R6) proportion of sequenced cfDNA nucleic acid molecules supporting a phased single nucleotide variant with a nearby single nucleotide polymorphism, if any, wherein the single nucleotide variant and single nucleotide polymorphism are separated by at most 110 bp;
R7) frequency of single nucleotide variant in the Catalogue Of Somatic Mutations In Cancer (COSMIC).
R8) predicted functional impact.

In one embodiment, a ranking in descending log odds likelihood order for the plurality of candidate somatic single nucleotide variant positions is followed by a selection of the top 40 for subsequent analysis. In other embodiments of the method, the top 20, or 30, or 40, or 50, or 60, or 70, or 80, or 90 candidate somatic single nucleotide variant positions are selected. This is the last part of the first step of the method (step A).

The second step (step B) comprises the same DNA sequencing library, wherein, in one embodiment, said DNA sequencing library is hybridized to a smaller pool of TAC oligonucleotides designed to capture genomic regions spanning the selected top 40 candidate somatic single nucleotide variant positions from Step A to obtain an enriched library and further wherein said enriched library is amplified and sequenced to obtain at least 200,000 cfDNA nucleic acid sequences per targeted region. In other embodiments of the method, a sequencing DNA library is also prepared for the corresponding buffy coat and is hybridized with the selected pool of TAC oligonucleotides, and sequenced to obtain at least 100,000 or 200,000 or 300,000 or 400,000 or 500,000 cfDNA nucleic acid molecules per targeted region. A classification model is used to determine whether a candidate somatic single nucleotide variant from the plurality of the list of selected candidate somatic single nucleotide variants is present in the sample, after the removal, if any, of variants due to clonal hematopoiesis. The classification model is an ensemble learning method. In one embodiment the method is Decision Random Forest. In other embodiments, the method is a Supporting Vector Machine or a Naive Bayes Classifier, or a logistic regression model or a Neural Network. The classification method is trained using at least 7,000,000 aligned cfDNA nucleic acid molecules from both normal reference samples not previously diagnosed with cancer and cancer patients with known somatic variants previously characterized using at least one tissue specimen. The said classification model comprises at least one of the following covariates:
C1) number of cfDNA nucleic acid molecules in which the variant allele is present;
C2) the average mapping quality of sequenced cfDNA nucleic acid molecules, relative to the reference genome sequence;
C3) ratio of good quality to poor quality sequenced cfDNA nucleic acid molecules, that support the variant;
C4) proportion of sequenced cfDNA nucleic acid molecules supporting a phased single nucleotide variant with a nearby single nucleotide polymorphism, if any, wherein the single nucleotide variant and single nucleotide polymorphism are separated by at most 110 bp;
C5) frequency of single nucleotide variant in the Catalogue Of Somatic Mutations In Cancer (COSMIC).

The output of the classifier is the estimate class/status for each candidate somatic single nucleotide variant, i.e., detected/not detected. The presence of cell-free tumor DNA nucleic acid molecules in the sample is determined by applying the following rule: if more than 5% of the plurality of the selected candidate somatic single nucleotide variants are detected in the targeted re-sequencing experiment then the sample is considered positive (i.e., cell-free tumor DNA nucleic acid molecules are detected in the sample), otherwise the sample is considered negative (i.e., cell-free tumor DNA nucleic acid molecules are not detected in the sample). In other embodiments of the method, the presence of cell-free tumor DNA nucleic acid molecules is determined if more than 1% or 10%, or 15% or 20% or 25% of the plurality of single nucleotide variants selected in Step A are detected in Step B.

In another embodiment, the step B is performed from a second sequencing library prepared form the same human sample.

Phasing of candidate somatic single nucleotide variants with germline heterozygous single nucleotide polymorphisms

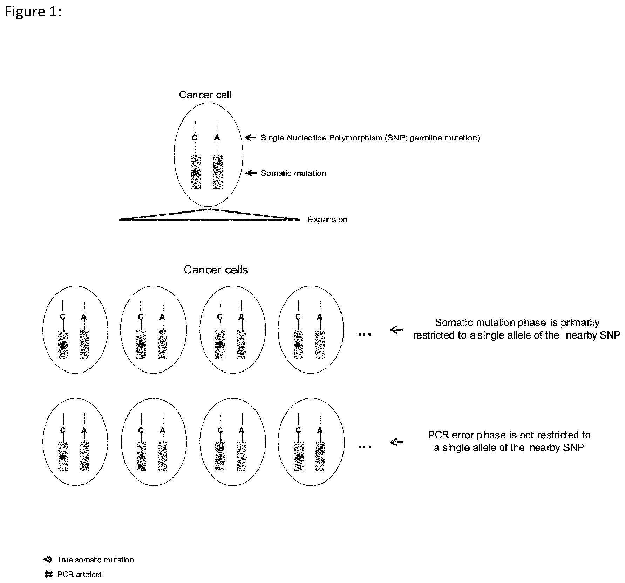

Phasing candidate somatic single nucleotide variants with germline heterozygous single nucleotide polymorphisms provide a powerful method for artifact elimination (Figure 1). In one embodiment of the method, heterozygous germline single nucleotide polymorphisms (SNPs) are detected in a sample using the plurality of aligned sequencing reads (cfDNA molecules), if the variant allele frequency at the genomic position lies between 0.3 and 0.7, and a Binomial test (on the variant counts with probability of success = 0.5) had p-value > 0.001. Thereafter, for each SNP a list of candidate somatic single nucleotide variant positions is created. Said list comprises candidate somatic single nucleotide variants in a distance less than or equal to 110bp so that a single aligned sequencing read can span both genomic positions and at least 3 aligned sequencing reads span both genomic positions. A proprietary algorithm (Python v2.7) calculates iteratively for each detected SNP with a non-empty said list: (a) the ratio of aligned sequencing reads that support the variant allele at the candidate somatic single nucleotide variant position and the reference allele at the SNP position over the total number of aligned sequencing reads that span both the SNP and the candidate somatic single nucleotide variant or (b) the ratio of aligned sequencing reads that support the variant allele at the candidate somatic single nucleotide variant position and the alternate/variant allele at the SNP position. A score H is defined as the maximum of (a) and (b). In one embodiment of the method a candidate somatic single nucleotide variant is filtered out if H is less than 0.95. In other embodiments of the method a candidate somatic single nucleotide variant is filtered out if H is less than 0.9 or 0.8.

### Example 1

In one embodiment of the method, a DNA sequencing library for each of five Seraseq reference samples comprising 29 single nucleotide variants observed at very low variant allele frequencies (VAFs) between 0.02%-0.3% (sample A: 7 single nucleotide variants at VAFs between 0.02-0.07%, sample B: 12 single nucleotide variants at VAFs between 0.1-0.3%, sample C: 5 single nucleotide variants at VAFs between 0.08-0.15%, sample D: 5 single nucleotide variants at VAFs between 0.2-0.7%, sample E: wild-type negative control) is prepared. The sequencing library for each sample includes unique molecular identifiers to uniquely tag each molecule. The said DNA sequencing libraries are subject to in-solution hybridization using a pool of TAC oligonucleotides with average size of 250 bp spanning a total of 500,000 bp of the human reference genome build 19 (hg19). The pool of TAC oligonucleotides is enriched with known hotspot regions of somatic single nucleotide variants in non-small cell lung cancer (adenocarcinoma and squamous cell carcinoma) tissue specimens. The estimated median mutation rate for stage I-III lung cancer tissues is 9/Mb (range: 7-13/Mb) (van de Heuvel et.al [2021], Respir Res 22: 302; The Caner Genome Atlas Program (TCGA); https://www.cancer.gov/ccg/research/genome-sequencing/tcga). The enriched libraries are sequenced at, at least, 5000X unique average depth resulting in sequencing cost of 20Gb on a Novaseq sequencing machine. A pipeline for alignment and consensus sequence generation for error suppression was prepared using samtools, bwa mem and fgbio bioinformatics suites. An ultra-sensitive variant calling was applied, i.e., even if at least one read supported a variant allele (and at most 25% to avoid germline SNPs), the substitution is selected as a candidate true somatic single nucleotide variant position. A background artifact model was applied to remove erroneous calls with a very high probability of being a false positive call (not a true somatic variant). Said background model estimated the number of times each false substitution (artifact) is present in the cohort of normal reference samples and the distribution of variant allele frequencies for each said artifact/erroneous call. Very common artifacts were removed/filtered-out. In one embodiment, a common artifact was defined as the one which is present in more than 5% of the normal reference samples. The list of candidate somatic variants was further reduced by removing the candidates with zero mapping quality, or a ratio of good quality to poor quality aligned cfDNA nucleic acid molecules that support the variant below a predefined threshold. In one embodiment, the predefined threshold was 2. The list of candidate somatic variants was further reduced by removing the candidate somatic single nucleotide variants that lie within a distance of less than 110 bp from at least one single nucleotide polymorphism detected in the sample and fail to be phased with the at least one single nucleotide polymorphism. A trained logistic regression model using the covariates R1-R7 is used to compute the log odds of each candidate somatic variant to be true and the top 40 candidates with the highest odds were selected for targeted re-sequencing. After alignment and consensus sequence generation using at least 200,000 raw read depth per targeted region, a trained decision random forest classification model with C1-C5 as covariates computed the number of detected variants in each sample. A flow chart illustrating the main steps of this embodiment of the invention is shown in Figure 2.

Sample A achieved a sensitivity of 86% (6 out of 7 true somatic variants detected; 95% CI: 42-99.6%), sample B achieved a sensitivity of 100% (12/12; 95% Cl: 74-100%), sample C achieved a sensitivity of 100% (5/5; 95% Cl: 48-100%), and sample D achieved a sensitivity of 100% (5/5; 95% Cl: 48-100%). For sample E, the wild-type negative control, none of the known variants were detected.

### Example 2

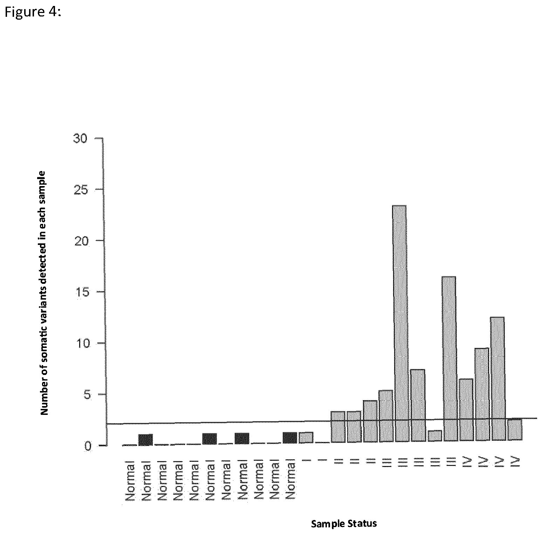

In another embodiment of the method, a DNA sequencing library is prepared for each of 11 normal reference samples taken from healthy donors and 14 cancer samples (NSCLC) taken from patients at stages I-IV (2 stage I, 3 stage II, 5 stage III and 4 stage IV) with an unknown number of single nucleotide variants. The sequencing library for each sample includes unique molecular identifiers to uniquely tag each molecule. Said DNA sequencing libraries are subject to in-solution hybridization using a pool of TAC oligonucleotides with average size of 250 bp spanning a total of 500,000 bp of the human reference genome build 19 (hg19). In one embodiment, the pool of TAC oligonucleotides is enriched with known hotspot regions of somatic single nucleotide variants in lung cancer (adenocarcinoma and squamous cell carcinoma) tissue specimens. The enriched libraries are sequenced at, at least, 3000X unique average depth resulting in sequencing cost of 20Gb on a Novaseq machine. A pipeline for alignment and consensus sequence generation for error suppression was prepared using samtools, bwa mem and fgbio suites. An ultra-sensitive variant calling was applied, i.e., even if at least one read supported a variant allele (and at most 25% to avoid germline SNPs), the substitution is selected as a candidate true somatic single nucleotide variant. Very common artifacts were removed/filtered-out. In one embodiment, a common artifact was defined as the one which is present in more than 5% of the normal reference samples. The list of candidate somatic variants was further reduced by removing the candidates with zero mapping quality or a ratio of good quality to poor quality aligned cfDNA nucleic acid molecules that support the variant below a pre-defined threshold. In one embodiment, the predefined threshold was 2. The list of candidate somatic variants was further reduced by removing the candidate somatic single nucleotide variants that lie within less than 110bp from at least one single nucleotide polymorphism detected in the sample and fail to be phased with the at least one single nucleotide polymorphism. A trained logistic regression model using covariates R1-R7 is used to compute the log odds of each candidate somatic variant to be true and the top 40 candidates with the highest odds were selected for targeted re-sequencing. A sequencing DNA library was also prepared for the buffy coat of all samples and hybridized with the selected pool of TAC oligonucleotides, sequenced and analyzed with the same pipeline to remove variants due to clonal hematopoiesis. Then, a trained decision Radom Forest classifier (covariates C1-C5) calculated the number of detected variants per sample. The results are shown in Figure 4. The y-axis represents the number of somatic variants detected in each sample. The black bars correspond to normal samples and the grey bars correspond to abnormal samples. The x-axis denotes the status of each sample (Normal or stage of cancer). The threshold to call a sample positive is represented by a horizontal solid black line. The clinical specificity of the test was 100% (11/11; 95% Cl: 72-100%) with a clinical sensitivity of 71% (10/14; 95% CI: 42-92%).

In another aspect, the invention provides kits for carrying out the methods of the disclosure. In one embodiment, the kit comprises a container consisting of the pool of TAC oligonucleotides and instructions for performing the method. In one embodiment, the TAC oligonucleotides are provided in a form that allows them to be bound to a solid support, such as biotinylated TAC oligonucleotides. In another embodiment, the TAC oligonucleotides are provided together with a solid support, such as biotinylated TAC oligonucleotides provided together with streptavidin-coated magnetic beads.

In one embodiment, the kit comprises a container comprising the pool of TAC oligonucleotides and instructions for performing the method, wherein the pool of TAC oligonucleotides comprises a plurality of TAC oligonucleotide families, wherein each TAC oligonucleotide family comprises a plurality of member sequences, wherein each member sequence binds to the same genomic sequence of interest but has different start and/or stop positions with respect to a reference coordinate system for the genomic sequence of interest, and further wherein:
(i) each member sequence within each TAC oligonucleotide family is between 100-500 base pairs in length, each member sequence having a 5' end and a 3' end;
(ii) each member sequence binds to the same genomic sequence of interest at least 50 base pairs away, on both the 5' end and the 3' end, from regions harboring Copy Number Variations (CNVs), Segmental duplications or repetitive DNA elements; and
(iii) the GC content of the pool of TAC oligonucleotides is between 19% and 80%, as determined by calculating the GC content of each member within each family of TAC oligonucleotides.

Furthermore, any of the various features described herein with respect to the design and structure of the TAC oligonucleotides can be incorporated into the TAC oligonucleotides that are included in the kit.

In various other embodiments, the kit can comprise additional components for carrying out other aspects of the method. For example, in addition to the pool of TAC oligonucleotides, the kit can comprise one or more of the following (i) one or more components for isolating cell-free DNA from a biological sample; (ii) one or more components for preparing the sequencing library (iii) one or more components for amplifying and/or sequencing the enriched library; and/or (iv) software for performing statistical analysis.

Preferably the kit comprises the TAC oligonucleotides of step A and B, wherein the TAC oligonucleotides of step B are a subset of the TAC oligonucleotides of step A.

## Claims

1. The invention relates to an in-vitro method of detecting a somatic mutation in a human,
(i) providing for a human sample, preferably a blood sample from a subject, more preferably a plasma sample, a serum sample, or a buffy coat sample,
(ii) preparing a first nucleic acid sequencing library from the nucleic acids present in said sample,
(iii) hybridizing one or more TAC oligonucleotides from a first TAC oligonucleotide pool (TAC-oligonucleotide 1) to said first nucleic acid library thereby isolating a first subset of library nucleic acid molecules,
(iv) sequencing said first subset of library nucleic acid molecules and comparing the sequences determined to a human reference sequence, thereby creating a first group of putatively informative sequence variants (PISV1),
(v) hybridizing one or more TAC oligonucleotides from a second TAC oligonucleotide pool comprising TAC oligonucleotides (TAC oligonucleotide 2) which are specific for the nucleic acid molecules of said first group of putatively informative sequence variants, to said first library of nucleic acid molecules thereby isolating a second subset of library nucleic acid molecules,
(vi) sequencing said second subset of library nucleic acid molecules and comparing the sequences determined to a human reference sequence, thereby creating a second group of putatively informative sequence variants (PISV2),
(vii) analyzing the putatively informative sequence variants (PISV2) thereby detecting a somatic mutation in the sample from the subject.

2. Method according to claim 1, wherein the second TAC oligonucleotide pool (TAC oligonucleotide-2) is a subset of the first TAC oligonucleotide pool.

3. Method according to claims 1 or 2, wherein each sequence in said first group of putatively informative sequence variants (PISV1) is ranked according to information value based on likelihood statistics.

4. The method of claims 1 to 3, wherein each sequence in said first group of putatively informative sequence variants (PISV1) is ranked according to information value based on likelihood statistics which is an ensemble learning method and wherein said method comprises:
(i) number of cfDNA nucleic acid molecules in which the variant allele is present;
(ii) the average mapping quality of sequenced cfDNA nucleic acid molecules, relative to the reference genome sequence;
(iii) ratio of good quality to poor quality sequenced cfDNA nucleic acid molecules, that support the variant;
(iv) proportion of sequenced cfDNA nucleic acid molecules supporting a phased single nucleotide variant with a nearby single nucleotide polymorphism, if any, wherein the single nucleotide variant and single nucleotide polymorphism are separated by at most 110 bp;
(v) frequency of single nucleotide variant in a disease specific database.

5. Method according to claims 1 to 4, wherein the TAC oligonucleotides of said first TAC oligonucleotide pool (TAC oligonucleotide 1) and said second TAC oligonucleotide pool (TAC oligonucleotide 2) are between 150 and 260 base pairs in length, wherein they are designed to bind a region of interest and wherein they have a 5' end and a 3' end.

6. Method according to claims 1 to 5, wherein the GC content of the pools of TAC oligonucleotides is between 19% and 80%, as determined by calculating the GC content of each member within the pool of TAC oligonucleotides.

7. Method according to claims 1 to 6, wherein the pool of TAC oligonucleotides comprise a plurality of TAC oligonucleotide families each directed to a different region of interest, wherein each TAC oligonucleotide family comprises a plurality of member sequences, wherein each member sequence binds to the same region of interest but has different start and/or stop positions with respect to a reference coordinate system for the regions of interest, and further wherein the start and/or stop positions for the member sequences within a TAC oligonucleotide family, with respect to a reference coordinate system for the regions of interest are staggered by 5 to 10 base pairs.

8. Method according to claims 1 to 7, wherein the second TAC oligonucleotide pool (TAC oligonucleotide 2) comprises a defined fraction of the first TAC oligonucleotide pool (TAC oligonucleotide 1) and it makes up 0.1%, or 0.25%, or 0.5%, or 0.75% or 1.0% but most preferably about 0.5% of said first TAC oligonucleotide pool (TAC oligonucleotide 1).

9. Method according to any of the previous claims wherein, the pool of TAC oligonucleotide 2 is designed to capture regions spanning the selected top 20, or 30, or 40, or 50, or 60, or 70, or 80, or 90 candidate somatic single nucleotide variant positions from Step A.

10. Method according to any of the previous claims wherein, step vi) of claim 1 comprises amplifying and sequencing said second subset of library nucleic acid molecules to obtain at least 200,000 cfDNA nucleic acid sequences per targeted region.

11. Method according to any of the previous claims wherein, apart from the serum sample the buffy coat fraction of the blood is analyzed in order to determine the fraction of sequence that have arisen due to clonal hematopoiesis.

12. The method of claim 1, wherein the human sample is a blood sample, or a serum sample, or a buffy coat sample, or a urine sample, or a sputum sample, or a ascitic fluid sample, or a cerebrospinal fluid sample or a pleural effusion sample or a saliva sample, or a bronchoalveolar lavage fluid, or an aspiration fluid sample from different parts of the body.

13. The method of claim 1, where in the human sample is a tissue sample or a stool sample.

14. The method of claim 1, wherein the method is used for the detection of donor-derived cell-free DNA.

15. The method of claim 1, wherein the alignment in step B is performed using the consensus sequence specific to the patient generated from step A.

16. The method of any of the preceding claims wherein the single nucleotide variant and single nucleotide polymorphism are separated by at most by 100 bp, or 120 bp or 130 bp, or 140 bp, or 150 bp.
